# EUROPÄISCHE PATENTANMELDUNG

(11) **EP 0 671 152 A1**
(43) Veröffentlichungstag der Anmeldung: **13.09.1995**
(21) Anmeldenummer: 95102221.9
(22) Anmeldetag: 17.02.1995
(51) Int. Cl.: A61B 19/10

(54) **Medizinisches Abdecktuch**

(30) Priorität: 12.03.1994 DE 9404219 U
(71) Anmelder: VYGON GMBH & CO KG, D-52070 Aachen (DE)
(72) Erfinder: Heiliger, Raymund, Dr., D-52134 Herzogenrath (DE); Jansen, Matthias Wilhelm, D-52223 Stolberg (DE); Oldenburg, Jens, D-52134 Herzogenrath (DE)
(74) Vertreter: Bauer, Hubert, Dipl.-Ing.

(57) **Zusammenfassung**

Um ein medizinisches Abdecktuch aus einem flüssigkeitsundurchlässigen, jedoch saugfähigem Material wie Zellstoff, Papier oder Kunststoff so auszubilden, daß es unter Beibehaltung seiner konventionellen Funktionen unabhängig von seiner Positionierung eine ordnungsgemäße Aufnahme für Medical-Produkte und -Instrumente bietet und dabei eine sichere Einhaltung der gewählten Aufnahmeanordnung gewährleistet, wird vorgeschlagen, an dem Abdecktuch mindestens ein von einer Tuchseite aus zugängliches, Medical-Produkte und/oder Einmalinstrumente aufnehmendes taschenförmiges Behältnis (6, 7) und/oder laschenförmiges Befestigungsteil (6a) vorzusehen.

## Beschreibung

Die Erfindung betrifft ein medizinisches Abdecktuch aus einem flüssigkeitsundurchlässigen, jedoch saugfähigen Material, wie Zellstoff, Papier oder Kunststoff.

Derartige allgemein bekannte Abdecktücher werden insbesondere bei medizinischen Operationen benötigt, um im ausgebreiteten Zustand das unmittelbare Umfeld eines Eingriffsbereichs abzudecken und/oder in dessen Nähe eine sterile Ablagefläche für Medical-Produkte ebenso wie für Operationsinstrumente zu schaffen. Die Abdecktücher müssen daher ebenso steril in Vorrat gehalten werden wie die Medical-Produkte und die Operationsinstrumente. Letztere stellen häufig sogenannte Einmalinstrumente dar, die bis zu ihrem Einsatz in steriler Verpackung bereitgehalten werden. Zur Vorbereitung bestimmter Operationen ist ein häufig übereinstimmendes Sortiment von sterilen Utensilien auszupacken und im ausgepackten Zustand vor einer Kontaminierung geschützt schnell und einfach zugänglich bereitzuhalten. Dazu bieten sich die bekannten Abdecktücher an, auf welche die Operationsutensilien verteilt plaziert werden können, sofern für die Tücher eine horizontale Auflagefläche verfügbar ist, die ihre Lage nicht verändert. Aber auch bei dieser Anordnung der Tücher ist nicht gewährleistet, daß die während einer
Operation benötigten Medical-Produkte und Einmalinstrumente ihre gewählte Plazierung hinreichend sicher beibehalten. In Unordnung geratene Operationsutensilien erschweren nicht nur den sicheren Zugriff, sondern müssen im Einzelfall sogar durch neue Teile ersetzt werden, sofern sich das eine oder andere Teil unbeabsichtigt außerhalb des sterilen Ablagebereichs verlagert hat.

Der Erfindung liegt die Aufgabe zugrunde, ein medizinisches Abdecktuch der eingangs beschriebenen Art so auszubilden, daß es unter Beibehaltung seiner konventionellen Funktionen unabhängig von seiner Positionierung eine ordnungsgemäße Aufnahme für Medical-Produkte und -Instrumente bietet und dabei eine sichere Einhaltung der gewählten Aufnahmeanordnung gewährleistet.

Zur Lösung dieser Aufgabe wird von einem medizinischen Abdecktuch der im Oberbegriff des Anspruchs 1 genannten Art ausgegangen, welches erfindungsgemäß die im kennzeichnenden Teil des Anspruchs 1 angegebenen Merkmale aufweist.

Durch das erfindungsgemäße taschenförmige Behältnis und/oder das laschenförmige Befestigungsteil als flacher Bestandteil des medizinischen Abdecktuches bietet sich die Möglichkeit, darin bzw. damit verschiedene Medical-Produkte und -Instrumente unterzubringen bzw. zu befestigen, die z.B. innerhalb des in einzelne Fächer unterteilbaren Behältnisses in einer bestimmten Anordnung sicher plazierbar sind und sich mühelos daraus entnehmen lassen. Sämtliche für eine bestimmte Operation benötigte Medical-Produkte und -Instrumente lassen sich auf diese Weise, ohne im einzelnen steril verpackt sein zu müssen, durch das Abdecktuch unmittelbar insgesamt steril verpacken, so daß lediglich das entsprechend bestückte Abdecktuch im gefalteten und/oder zusammengerollten Zustand einer sterilen Gesamtverpackung bedarf. Unmittelbar vor einer Operation wird diese Verpackung geöffnet und das medizinische Abdecktuch mit seinen darin integrierten Utensilien entnommen, ausgebreitet und mit seiner die Öffnung zu der Tasche aufweisenden Seite zugänglich plaziert.

Nach Ausgestaltungen der Erfindung besteht das Behältnis und das Befestigungsteil aus dem gleichen Material wie das Abdecktuch, wobei das Behältnis wie eine Tasche und das Befestigungsteil mit seinen beiden Enden wie eine Lasche in einem Bekleidungsstück in das Abdecktuch eingearbeitet oder aber auch auf das Abdecktuch aufgesetzt sein kann.

Statt ein Behältnis in mehrere Fächer zu unterteilen, läßt sich der gleiche Zweck auch dadurch erzielen, daß nach Ausgestaltungen der Erfindung mehrere Behältnisse und Befestigungsteile in einer Reihe nebeneinander oder auch übereinander angeordnet werden, wobei auch mehrere Reihen von Behältnissen und Befestigungsteilen über die Gesamtfläche des Abdecktuches verteilt angeordnet werden können, um die Gesamtfläche des Abdecktuches auch insoweit zu nutzen.

Nach einer besonders vorteilhaften Ausgestaltung der Erfindung sind mindestens einzelne Behälter mit einer flüssigkeitsdichten Auskleidung versehen, so daß diese von Operationsutensilien befreiten Behältnisse auch zur Aufnahme von überschüssigen Medikamenten, abgesaugten Sekreten und dergleichen dienen können.

Eine in ihrer Bedeutung außerordentlich wesentliche Ausgestaltung der Erfindung besteht darin, mindestens einige Behältnisse mit einem Einsatz zu versehen, in den Instrumente wie insbesondere Nadeln mit ihrer Spitze einstechbar und verletzungsfrei plazierbar sind. Die Verletzungs- und Infektionsgefahr durch kontaminierte Nadeln wird inzwischen von der Fachwelt als besonders hohes Risiko für Mediziner und Krankenhauspersonal bis hin zu den mit der Entsorgung befaßten Personen angesehen. Dieser Gefahr begegnet der erfindungsgemäße Einsatz in sehr wirkungsvoller Weise, indem dessen Materialzusammensetzung einerseits hinreichend elastisch ist, um darin eine Nadelspitze einzustechen, andererseits aber zum Beispiel durch eine nicht durchstechbare Verhautung auf der der Einstechseite gegenüberliegenden Seite einen Austritt der Nadelspitze verhindert und schließlich auch die Nadel hinreichend fixiert.

Zur freien Plazierbarkeit des Abdecktuches sieht eine Ausgestaltung der Erfindung schließlich noch vor, mindestens an einem oberen Randstreifen des Abdecktuches Selbstklebestreifen oder Halteklammern anzubringen, so daß sich das Abdecktuch mit seiner Ebene insbesondere auch vertikal ausrichten läßt, wobei lediglich noch darauf zu achten ist, daß die einheitlich ausgerichteten Behältnisöffnungen nach oben weisen.

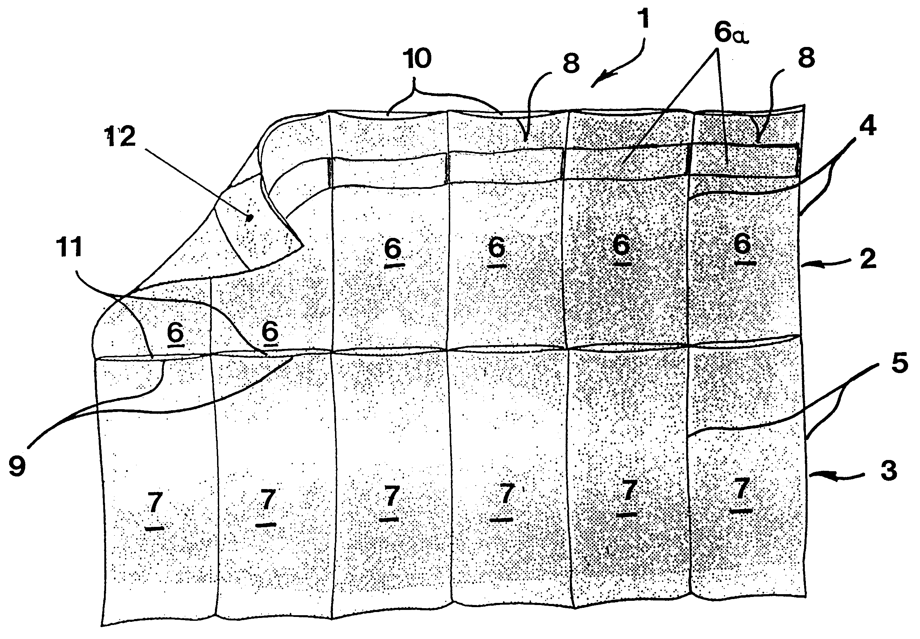

In der Zeichnung ist ein Ausführungsbeispiel eines erfindungsgemäßen Abdecktuches schematisch dargestellt:
Ein aus Zellstoff hergestelltes rechteckförmigens Abdecktuch 1 ist in ein oberes Feld 2 und ein unteres Feld 3, die im wesentlichen die gleiche Größe aufweisen, unterteilt. Die Felder 2 und 3 sind ihrerseits jeweils in sechs Feldabschnitte 4 bzw. 5 aufgeteilt.

Jeder Feldabschnitt 4 und 5 ist doppelwandig ausgebildet, so daß taschenförmige Behältnisse 6 und 7 entstehen, an deren jeweiliger Oberseite durch einen Einschnitt 8 bzw. 9 eine Öffnung 10 bzw. 11 gebildet ist.

Die obere Reihe der taschenförmigen Behältnisse 6 ist in ihrem oberen Drittel zusätzlich mit laschenförmigen Befestigungsteilen 6a versehen, deren Enden jeweils mit der Außenseite der Behältnisse 6 verbunden sind.

Auf der den Öffnungen 10, 11 abgewandten Seite des Abdecktuches sind Befestigungsmittel angebracht. Wie der umgeschlagene obere linke Eckbereich des Abdecktuches 1 erkennen läßt, bestehen diese Befestigungsmittel aus Klebestreifen 12, die in gleichmäßiger Verteilung entlang der Oberkante angeordnet sein können. Weitere Selbstklebestreifen können darüber hinaus auf der Rückseite des Abdecktuches so positioniert sein, daß sich mit deren Hilfe das Abdecktuch 1 im gefalteten und/oder zusammengerollten Zustand als kompakte, die erforderlichen Operationsutensilien einschließende Einheit darstellt.

## Patentansprüche

1. Medizinisches Abdecktuch aus einem flüssigkeitsundurchlässigen, jedoch saugfähigen Material, wie Zellstoff, Papier oder Kunststoff, gekennzeichnet, durch mindenstens ein von einer Tuchseite aus zugängliches, Medical-Produkte und/oder Einmalinstrumente aufnehmendes taschenförmiges Behältnis (6, 7) und/oder laschenförmiges Befestigungsteil (6a).

2. Abdecktuch nach Anspruch 1, dadurch gekennzeichnet, daß das Behältnis (6, 7) und das Befestigungsteil (6a) aus dem gleichen Material wie das Abdecktuch (1) bestehen.

3. Abdecktuch nach Anspruch 1 oder 2, dadurch gekennzeichnet, daß das Behältnis (6, 7) und das Befestigungsteil (6a) in das Abdecktuch (1) eingearbeitet oder auf das Abdecktuch (1) aufgesetzt ist.

4. Abdecktuch nach einem der Ansprüche 1 bis 3, dadurch gekennzeichnet, daß mehrere Behältnisse (6, 7) und Befestigungsteile (6a) in einer Reihe nebeneinander oder übereinander angeordnet sind.

5. Abdecktuch nach einem der Ansprüche 1 bis 4, dadurch gekennzeichnet, daß mehrere Reihen von Behältnissen (6, 7) und Befestigungsteilen (6a) über die Grundfläche des Abdecktuches (1) verteilt angeordnet sind.

6. Abdecktuch nach einem der Ansprüche 1 bis 5, dadurch gekennzeichnet, daß mindestens einzelne Behältnisse (6, 7) mit einer flüssigkeitsdichten Auskleidung versehen sind.

7. Abdecktuch nach einem der Ansprüche 1 bis 6, dadurch gekennzeichnet, daß mindestens einzelne Behältnisse (6, 7) mit einem Einsatz versehen sind, in den Instrumente, wie Nadeln, mit ihren Spitzen einstechbar und verletzungsfrei plazierbar sind.

8. Abdecktuch nach einem der Ansprüche 1 bis 7, gekennzeichnet durch an mindestens einem oberen Randstreifen des Abdecktuches (1) angebrachte Selbstklebestreifen (12) oder Halteklammern.
